# EUROPEAN PATENT APPLICATION

(11) **EP 2 550 981 A1**
(43) Date of publication of application: **30.01.2013**
(21) Application number: 11759310.3
(22) Date of filing: 17.03.2011
(51) Int. Cl.: A61L 27/00, A61B 17/58, A61C 8/00, A61F 2/28, A61F 2/30

(54) **METAL MATERIAL FOR BIOIMPLANT**

(30) Priority: 23.03.2010 JP 2010065515
(71) Applicant: Kyocera Medical Corporation, Osaka-shi, Osaka 532-0003 (JP)
(72) Inventor: ISHIAI, Akira, Osaka-shi Osaka 532-0003 (JP); ISHIDA, Noriyuki, Osaka-shi Osaka 532-0003 (JP); SHIBATA, Fusako, Osaka-shi Osaka 532-0003 (JP); MAENO, Sumihiko, Osaka-shi Osaka 532-0003 (JP)
(74) Representative: Müller-Boré & Partner Patentanwälte
(86) International application number: PCT/JP2011/056452
(87) International publication number: WO 2011/118504

(57) **Abstract**

A metal material for a bioimplant which is excellent in biointegration according to the present invention has a surface treated by sandblasting, and
the surface has an average roughness Ra of 1 to 2.5 µm and is made free from residual abrasive used in the sandblasting treatment by washing with water after the sandblasting treatment and the composition of the metal material surface does not change before and after the sandblasting treatment.

According to the present invention, it is possible to provide a bioimplant metal material from which an abrasive used for sandblasting treatment can be easily removed by washing with water and which is excellent in biointegration to a bone or the like since having a good roughened surface and a production method thereof.

## Description

### TECHNICAL FIELD

The present invention relates to a metal material for a bioimplant, a production method thereof, and an implant, and more particularly to a metal material for a bioimplant to be used for an implant to be implanted in a bone.

### BACKGROUND ART

In order to improve integration (integration strength) to a bone, a metal material for a bioimplant to be implanted in a living body, particularly in a bone is generally subjected to surface-roughening process to roughen at least a portion of a surface (surface brought into contact with a bone) of a part to be implanted in a bone. The surface-roughening process increases the surface area having contact with a bone and the bone enters in concave are (causing an anchor effect) to increase the integration strength to the bone. Titanium or a titanium alloy which is chemically stable and shows the most excellent biocompatibility among metal materials is widely used as the metal material. After the surface-roughening treatment, bioactivation treatment may be carried out in some cases in order to provide a direct bonding property to a bone (bioactivity). The bioactivation treatment may be an alkali- and heat-treatment as described in, for example, Patent Document 1.

Methods commonly employed as a surface-roughening method are a method for coating a surface of a metal material with an uneven layer by plasma spraying or arc spraying and a method for sandblasting a surface of a metal material by blast grains (blast sand). However, in the case of the former method, since fatigue strength of a metal material is generally lowered, the method is not employed for a portion bearing a heavy load. Although depending on treatment conditions, the latter sandblasting treatment can suppress decrease of fatigue strength, so that this treatment is applicable to a portion bearing a heavy load.

In general, a substance with high hardness is used as blast sand (abrasive) for the sandblasting treatment and α-alumina (α-phase aluminum oxide crystal, hereinafter, simply referred to as alumina) is widely used. However, in the case where alumina is used as blast sand, an alumina component remains on the surface of a metal material after the sandblasting treatment and is recognized as a bioinert material in a living body, so that there is a risk that adhesion to a bone could be delayed. Removal of alumina adhering to the surface is extremely difficult and it is impossible to remove alumina by common ultrasonic washing.

Because of this, techniques described in Patent Documents 2 and 3 are proposed as a sandblasting method that does not leave inactive impurities such as alumina.

Particularly, Patent Document 2 discloses a method of using sintered hydroxyapatite (HAP) or tricalcium phosphate, which is a material having high biocompatibility and absorbed in a bone, as blast grains. Patent Document 2 discloses that in this method, when sintered HAP particles are blown to a Ti core member and reach the Ti surface, extremely finely crushed particles stick the Ti core member and the stuck fine particles are not removed by common ultrasonic washing and remain; however, HAP has osteoconductivity and therefore contributes to bone formation immediately after implantation.

Patent Document 3 has been made in consideration of the problem of the technique disclosed in Patent Document 2 and discloses a sandblasting method, using a shot material (abrasive) containing fluoroapatite. That is, HAP disclosed in Patent Document 2 has problems, for example, it is easy to be decomposed at a high temperature of 1000°C or higher and is difficult to produce a dense sintered body and is also difficult to carry out surface roughening to give desired implant surface. In the meantime, fluoroapatite has characteristics that its crystal structure is dense and difficult to be decomposed at a high temperature and that it can be dissolved easily by an acid, although it is slightly inferior in biocompatibility as compared with HAP. Therefore, Patent Document 3 discloses that the method of this document can give good surface roughness and easily remove a remaining shot material by an acid.

### PRIOR ART DOCUMENTS

### PATENT DOCUMENTS

Patent Document 1: Japanese Patent No. 2775523
Patent Document 2: JP-A-10-99348
Patent Document 3: JP-A-2009-136632

### SUMMARY OF THE INVENTION

### PROBLEMS TO BE SOLVED BY THE INVENTION

As described above, the method disclosed in Patent Document 3 provides a sandblasting method by which a remaining abrasive can be removed by an acid; however, it is desired to provide a technique by which a remaining abrasive can be removed more conveniently only by carrying out washing with water and which is excellent biointegration to a bone or the like.

In view of the above-mentioned state of the art, an object of the present invention is to provide a novel bioimplant metal material from which an abrasive used for sandblasting treatment can be easily removed by washing with water and which is excellent in biointegration to a bone or the like since having a good roughened surface and a production method thereof.

### MEANS TO SOLVE THE PROBLEM

A metal material for a bioimplant which is excellent in biointegration according to the present invention which solves the above-mentioned problems is characterized in that the metal material for a bioimplant has a surface treated by sandblasting, and the surface has an average roughness Ra of 1 to 2.5 µm and is made free from residual abrasive used in the sandblasting treatment by washing with water after the sandblasting treatment and the composition of the metal material surface does not change before and after the sandblasting treatment.

In the preferable embodiment, the abrasive is borax.

In the preferable embodiment, the metal material is Ti or a Ti alloy.

In the preferable embodiment, the metal material is further treated by bioactivation treatment.

The present invention includes an implant obtained by using the above metal material for a bioimplant. The implant may include for example, a dental implant, an artificial joint, a member for bone joining, or an artificial bone made of a metal.

Also, a method for producing a metal material for a bioimplant according to the present invention which solves the above-mentioned problems is characterized in that the production method is processed sandblasting treatment of a surface of a metal material for a bioimplant by using borax.

In the preferable embodiment, bioactivation treatment is processed after the sandblasting treatment.

### EFFECTS OF THE INVENTION

According to the present invention, it is possible to provide a bioimplant metal material from which an abrasive used for sandblasting treatment can be easily removed by washing with water and which is excellent in biointegration since having a good roughened surface and a production method thereof.

### BRIEF DESCRIPTION OF THE DRAWINGS

[Fig. 1] Fig. 1 is a graph showing a result of EDX analysis of elements existing in a metal material surface after sandblasting treatment using borax in Example 1.
[Fig. 2] Fig. 2 is a graph showing a result of EDX analysis of elements existing in a metal material surface after sandblasting treatment using borax and alumina in Example 2.
[Fig. 3] Fig. 3 is a graph showing a result of investigation on an adhesion effect of a metal material to a bone after sandblasting treatment using borax and alkali- and heat-treatment in Example 3.

### MODE FOR CARRYING OUT THE INVENTION

Inventors of the present invention have made investigations particularly to provide an abrasive for sandblasting treatment in place of alumina in order to solve the problem (lowering of biointegration because of alumina residue) in sandblasting treatment using alumina. Consequently, the inventors of the present invention have found that by using borax as an abrasive, the problem of residue is not caused since the borax can be removed easily and completely by washing with water after sandblasting treatment and thus a metal material for a bioimplant excellent in biointegration as compared with the case of using alumina can be obtained, and the finding has now led to completion of the present invention.

As described above, the present invention is characterized in that borax is used as an abrasive to be used for sandblasting treatment.

Borax is a crystal of sodium tetraborate and has high water-solubility. Borax has been used as a raw material ore for boron and also as a detergent and a preservative by utilizing its washing function and sterilization function, but has low hardness as compared with alumina and thus has not been used as a blasting treatment material so far. According to the results of experiments made by the inventors of the present invention, it was found that borax can be easily removed by washing with water after blasting treatment and no residue is observed on the surface of a metal material.

Moreover, it was found that the surface of a metal material treated by sandblasting using borax has high adhesion to a bone although the surface roughness Ra is small as compared with that treated by sandblasting using alumina. That is, it is understood that although borax is inferior in the rough surface-effect (blast effect) as compared with alumina, borax can realize surface roughness to an extent of causing an adhesion effect (anchor effect) with a bone by the surface-roughness. Since neither lowering of the adhesion effect because of residues on the surface of a metal material nor lowering of fatigue strength is observed, it is supposed that borax can effectively cause the anchor effect as it is.

A metal material of the present invention is different from a metal material treated by sandblasting by using alumina in that no residue of the abrasive used in the sandblasting treatment is caused and the composition of the metal material does not change before and after the sandblasting treatment. In the present invention, "no residue of the abrasive used in the sandblasting treatment is caused" means that the remaining abrasive is removed by washing with water after sandblasting treatment and no peak of components derived only from the abrasive which exceeds a noise level is detected when the composition of the surface of a metal material is analyzed by EDX or the like. "The composition of the metal material does not change before and after the sandblasting treatment" means that the ratio of peak intensity of elements composing the metal material substantially does not change before and after the treatment in the case where the composition analysis of the surface of the metal material is analyzed by EDX or the like before the sandblasting treatment and after the treatment and washing with water (in the present invention, "after the treatment" means "after washing with water").

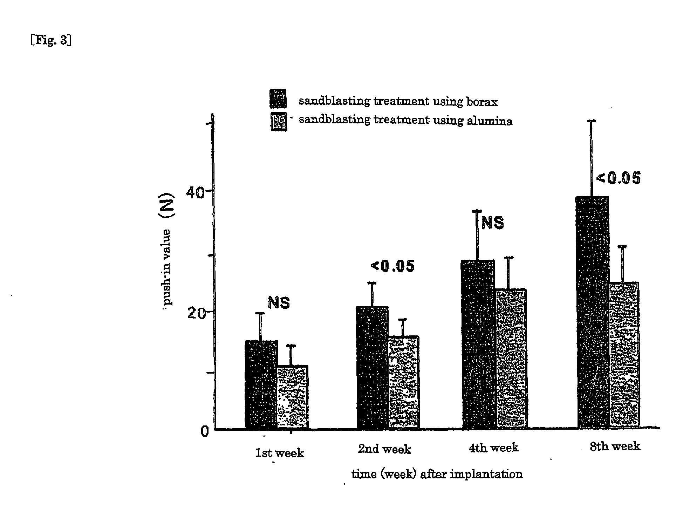

It was also found that if bioactivation treatment (representatively, the alkali- and heat-treatment disclosed in Patent Document 1 or the like) is carried out aiming at giving a direct bonding property to a bone (bioactivity) to the surface after sandblasting, joining to a bone after implantation is promoted more than in the case of using alumina to give earlier and stronger adhesion to the bone (see Fig. 3 explained later). The detailed reason for this is not made clear, but it is supposed that borax employed in the present invention can be completely removed by washing with water after sandblasting and does not remain on the metal material surface and thus, the bioactivation effect by the alkali- and heat-treatment can be effectively caused without being inhibited.

Consequently, borax employed in the present invention is remarkably useful as an abrasive for sandblasting for the surface of a metal material for an implant in place of widely used alumina and is highly expected as a dental implant, a stem of an artificial joint, and the like for which combination of material strength and bone adhesion are required.

Mainly in consideration of such as the balance between a desired anchor effect and efficiency of sandblasting treatment, it is preferable to properly control the particle diameter (the particle diameter range classified by sieves with different mesh sizes) of borax to be employed in the present invention. The particle diameter is preferably approximately in a range of 10 to 2000 µm. If the particle diameter is too small, it is impossible to obtain a desired anchor effect and besides, the fluidity is deteriorated so that a problem such as in jetting out of a blast apparatus is caused, resulting in lowering of the sandblasting treatment efficiency. On the other hand, if the particle diameter is too large, a jet nozzle of a sandblasting apparatus is clogged. The particle diameter is more preferably 280 to 600 µm (a range of meshes is from passing meshes of 28 to on meshes of 60).

Borax satisfying the above-mentioned particle diameter can be prepared by purchasing a commercialized product produced from a Japanese or foreign reagent manufacturer/distributor and properly crushing and classifying the product. Concretely, borax commercialized by, for example, Naito Shouten, Showa Chemical Industry Co., Ltd., Toyama Pure Chemical Industries Ltd., etc. can be used.

The average roughness Ra of the metal material surface treated by sandblasting using borax is about 1 to 2.5 µm and smaller than Ra of the metal material surface treated by sandblasting using alumina under the same condition (about 3 to 6 µm). Although the Ra is smaller in the present invention, the desired anchor effect (effect of improving the integration strength to a bone) by the sandblasting treatment is effectively caused and borax can be easily and completely removed by washing with water after sandblasting treatment, so that there is no risk of causing a bad effect on bioactivation treatment carried out subsequently. The above-mentioned range of Ra is obtained by processing common sandblasting treatment using borax and if the Ra is within the range, the desired anchor effect can be guaranteed.

This Ra is measured according to JIS B 0601 (2001) and JIS B 0633 (2001). The measuring speed is 1 mm/sec and the evaluation length is 12.5 mm (cut-off value 2.5 mm) or 4 mm (cut-off value 0.8 mm).

The type of a metal material to be used in the present invention is not particularly limited as long as it is one which can be used for an implant and examples thereof include pure Ti, a Ti alloy, stainless steel, a cobalt-chromium-molybdenum alloy, a zirconium alloy, tantalum and an alloy thereof or the like. In consideration of the mechanical properties such as strength and hardness as well as compatibility with a bone, pure Ti or a Ti alloy is preferably used. The type of a Ti alloy is also not particularly limited and examples thereof include Ti alloys containing at least one element such as Al, V, Zr, Mo, Nb, and Ta. Concrete examples thereof include a Ti-6 mass% Al-4 mass% V alloy, a Ti-15 mass% Mo-5 mass% Zr-3 mass% Al alloy, and a Ti-6 mass% Al-2 mass% Nb-1 mass% Ta-0.8 mass% Mo alloy or the like.

The present invention is characterized by use of borax, but the condition of sandblasting is not particularly limited and the condition may be set properly so that desired surface roughness can be obtained. Concretely, the sandblasting may be processed using borax with the above-mentioned particle diameter under the conditions of the jet pressure of 1 to 5 Kgf/cm², the distance from a nozzle of 1 to 20 cm, and the treatment time of 1 to 30 seconds or the like.

After sandblasting, washing with water is carried out to remove remaining borax. Since borax is water-soluble, it is easily removed by washing with water. Although the conditions differ depending on the type of such as a metal material to be used, the washing with water is preferably carried out, for example, at normal temperature for 10 minutes to 1 hour by using an ultrasonic washing apparatus.

A metal material surface-roughened in the above-mentioned manner is subjected to bioactivation treatment represented by alkali- and heat-treatment and accordingly, a layer to be directly joined to a bone is formed and stronger integration strength to the bone can be obtained. Concretely, the alkali- and heat-treatment is carried out by immersion in an alkaline solution such as an aqueous sodium hydroxide solution (concentration of 2 to 10 M) at a temperature of 40 to 70°C for about 2 to 48 hours, washing with water, drying, and subsequent heating at 300 to 800°C for 1 to 24 hours in atmospheric air. The alkali- and heat-treatment to be employed in the present invention can be carried out with reference to the method described in, for example, Patent Document 1.

A metal material obtained in the above-mentioned manner may be used preferably as an implant material for artificial joints such as artificial hip joints, artificial knee joints, and artificial shoulder joints in orthopedic surgery fields; members for joining bones such as bone screws, bone plates, and spine fixation materials; other artificial bones made of metals; and dental implants in dentistry fields.

### EXAMPLES

Hereinafter, the present invention will be described more specifically by way of Examples, but the present invention does not undergo restriction by the following Examples, the present invention can be also implemented by appropriate alteration in such a range that can be in conformity with the gist described above and later, and all of them are included in the technical scope of the present invention.

### Example 1

In this example, elements existing in a metal material surface after sandblasting treatment using borax were analyzed.

More specifically, a machine processed surface of a Ti-15 mass% Mo-5 mass% Zr-3 mass% Al alloy as a metal material was treated by sandblasting with borax (commercialized borax classified by passing through meshes of 28 and leaving on meshes of 60; particle diameter 280 to 600 µm) under conditions of a jet pressure of 2 to 3 Kgf/cm², a distance from the nozzle of 1 to 10 cm, and a treatment time of 1 to 10 seconds.

The surface roughness Ra of the metal material surface after the sandblasting was measured according to the above-mentioned method to find it was 2 µm.

The elements existing in the sample surface washed with water after the sandblasting treatment were analyzed by high resolution energy dispersive x-ray spectrometer (KEVEX, manufactured by Cambridge Corporation (Massachusetts, USA); detector Be window thickness 5 mm). The measurement condition was that the electron beam accelerating voltage was 20 kV and a spectrum was adjusted to obtain quantitative results.

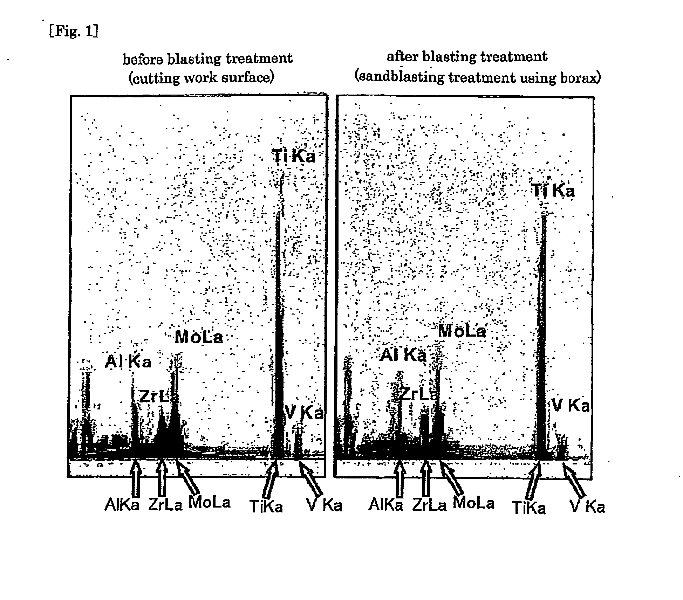

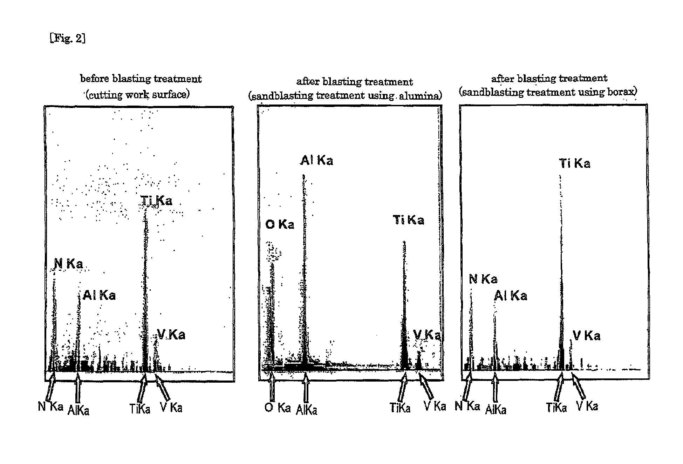

The surface element analysis before sandblasting treatment was carried out in the same manner for comparison. The results are shown in Fig. 1. Fig. 1 shows the results of an analysis chart by the above-mentioned analysis apparatus as it is, and element names corresponding to the respective peaks are also shown in the margin of Fig. 1 since the element names overlap at peak positions to make it difficult to understand. This is the same for Fig. 2 described later.

As shown in Fig. 1, the peak intensity of elements (Ti, Mo, Zr, Al) composing the metal material did not change before and after sandblasting. Further, since no peak derived from Na contained in borax was observed after sandblasting, it was revealed that the borax used as an abrasive is completely removed from the metal material surface by washing with water.

According to the above-mentioned experiment result, it was revealed that the metal material does not change before and after the sandblasting treatment and no residue of borax used in the sandblasting treatment is observed if sandblasting treatment is carried out using borax.

### Example 2

In this example, elements existing in the metal material surface were analyzed after sandblasting treatment was carried out for a Ti-6 mass% Al-4 mass% V alloy by using borax.

More specifically, a Ti-6 mass% Al-4 mass% V alloy was used as a metal material of a substrate and the metal material surface was measured in the same manner as in Example 1 after sandblasting treatment and washing with water carried out in the same manner as in Example 1.

For comparison, the metal material surface was measured after sandblasting treatment carried out in the same manner as blasting with borax and washing with water, except that commercialized blast sand made of α-alumina (particle size of 50 to 150 µm) was used in place of borax and the jet pressure was changed to about 6 kgf/cm². The surface element analysis before sandblasting treatment was also carried out. The results are shown in Fig. 2.

The surface roughness Ra of the metal material surface after the sandblasting was measured according to the above-mentioned method to find that the Ra was 2 µm in the case where borax was used and the Ra was 4 µm in the case where alumina was used.

As shown in Fig. 2, the peak intensity of elements (Ti, Al, V) composing the metal material did not change before and after sandblasting in the case where borax was used as a shot material.. Further, since no peak derived from Na contained in borax was observed after sandblasting, it was understood that the borax used as an abrasive is completely removed from the metal material surface by washing with water.

On the other hand, in the case where alumina was used, the peak intensity of Al was significantly increased as compared with that of Ti, a main component, after sandblasting. The high Al peak intensity was derived from alumina used as a shot material and it is supposed that alumina remained in the metal material surface.

According to the experiment result, it was understood that if sandblasting treatment is carried out using borax, the metal material does not change before and after the sandblasting treatment and no residue of the borax used in the sandblasting treatment was observed, and on the other hand, if alumina was used, Al in an amount derived from alumina remains in the metal material surface after treatment.

### Example 3

In this example, an adhesion effect of a metal material to a bone after sandblasting treatment using borax and alkali- and heat-treatment was investigated.

More specifically, a Ti-6 mass% Al-4 mass% V alloy same as the alloy used in Example 2 was used as a substrate, which was treated by sandblasting and washing with water and subsequently by alkali- and heat-treatment. The treatment method for the alkali- and heat-treatment was as follows. First, an aqueous NaOH solution with a concentration of 5 M (mole concentration) was prepared and the solution was heated and kept at a temperature of 60°C. The substrate subjected to sandblasting and washing with water was degreased with acetone, washed with distilled water and thereafter immersed in the solution for 24 hours. After the immersion, the substrate was taken out of the solution and subjected to washing treatment with distilled water for over a dozen minutes by using an ultrasonic washing apparatus. After properly dried, the substrate was put in an electric furnace and heated to 600°C in atmospheric air, kept for 1 hour, and cooled to room temperature. The sample produced in the above-mentioned manner was put in a sterilization bag, sterilized by gamma-ray, and subjected to an implantation experiment into a bone. The implantation experiment into a bone was carried out according to an experiment method described in a document (T. Ogawa et al., Biomechanical evaluation of osseous implants having different surface topographies in rats, J Dent Res, 79 (11), 1857-1863 (2000)) to investigate the adhesion effect of the metal material and the bone.

Concretely, each one specimen of the metal material (size: φ1 mm x L 2 mm) subjected to the above-mentioned treatment was implanted at a position of about 11 mm from the furthest end of the femur of an anesthetized rat (about 8 week-old) and at 1 week, 2 weeks, 4 weeks, and 8 weeks after the implantation, the specimen was taken out together with the femur and the bonding strength (push-in value) between the femur and the metal material was evaluated by a pushing test using an instron testing apparatus. For comparison, alumina blast sand same as in Example 2 was used in place of borax and the same experiment was carried out as described above.

The results are shown in Fig. 3. In Fig. 3, the transverse axis shows the time (week) after implantation and the vertical axis shows the bonding strength (N) to the bone. The graph shows the results of average value + standard deviation of each group with number of specimens n = 6 and in the graph, NS means no statistic significance with a critical rate of 5% and <0.05 means statistic significance with a critical rate of less than 5%.

It can be understood from Fig. 3 that, in the case where borax was used as a shot material, high integration strength to the bone was observed in all of the experiment periods as compared with the case where alumina was used. More specifically, it was revealed that the strong integration strength to the bone obtained on the 8th week after implantation in the case where alumina was used can be obtained within an extremely short time of 4th weeks or shorter after implantation in the case where borax was used. Consequently, borax employed in the present invention was confirmed to be remarkably useful as an abrasive for sandblasting for the surface of a metal material for an implant in place of widely used alumina.

The significant difference in the integration strength to the bone between both cases is supposedly attributed to the cleanness of the metal material surface after the sandblasting treatment. Since having high hardness, alumina is a remarkably preferable material as blast sand; however, alumina remains stuck to the metal material surface even after treatment and is hard to be removed. On the other hand, borax to be used in the present invention has relatively low hardness and is inferior in grinding effect as blast sand; however, borax reliably gives surface roughness adequate for causing a desired anchor effect and since being a water-soluble substance, the residue can be easily and completely removed by washing with water after the sandblasting and thus it is supposed to be possible to give strong adhesion to a bone.

According to the above-mentioned experiment results, it was proved that the metal material of the present invention obtained by using borax is extremely useful from the viewpoint that the strong adhesion to a bone can be obtained in an early stage.

The metal material of the present invention is preferably used for implants such as an artificial joint and a dental implant. For example, in an artificial hip joint stem, it is expected that quick adhesion to a bone can be achieved without lowering the strength of the stem of a type which is a thin stem with a square cross section and having no sprayed (porous) part and entirely subjected to blasting treatment in the implant region in the bone (known as European type). Further, although it is common to carry out surgeries twice in the case of a dental implant, since strong adhesion of an implant to a bone can be obtained in an early stage according to the present invention, it is possible to reduce the period (the period until the fixation of an upper structure) from the first surgery of stitching gingiva after implantation of an implant part (fixture) in a jawbone to the second surgery for installation of the upper structure (a tooth crown) after several months during which the fixture and the bone are sufficiently bonded. Further, since the metal material of the present invention has smaller surface roughness as compared with a metal material obtained using alumina, the strength of the stem itself can be maintained and it leads to a wide range of freedom of stem designing for shape conformability or the like. In case it becomes necessary to pull out a stem because of infection or the like after a surgery, a surgery is employed for separating of the bone and the stem by hitting a thin blade type bone chisel in the boundary between the stem and the bone. In this case, the metal material is advantageous in the point that the bone chisel is more easily inserted in and removed from the surface blasted by borax than the rough surface blasted with alumina sand.

## Claims

1. A metal material for a bioimplant having a surface treated by sandblasting and is excellent in biointegration, wherein
the surface has an average roughness Ra of 1 to 2.5 µm and is made free from residual abrasive used in the sandblasting treatment by washing with water after the sandblasting treatment and
the composition of the metal material surface does not change before and after the sandblasting treatment.

2. The metal material for a bioimplant according to claim 1, wherein the abrasive is borax.

3. The metal material for a bioimplant according to claim 1 or 2, wherein the metal material is Ti or a Ti alloy.

4. The metal material for a bioimplant according to any one of claims 1 to 3, wherein the surface is further treated by bioactivation treatment.

5. An implant obtained by using the metal material for a bioimplant according to any one of claims 1 to 4.

6. The implant according to claim 5 for a dental implant, an artificial joint, a member for bone joining, or an artificial bone made of a metal.

7. A method for producing an implant by processing sandblasting treatment of a surface of a metal material for a bioimplant by using borax.

8. The production method according to claim 7, wherein bioactivation treatment is processed after the sandblasting treatment.

9. The production method according to claim 8, wherein the bioactivation treatment is alkali- and heat-treatment.
